# EUROPEAN PATENT SPECIFICATION

(11) **EP 4 444 224 B1**
(45) Date of publication and mention of the grant of the patent: **02.09.2026**
(21) Application number: 22854271.8
(22) Date of filing: 21.12.2022
(51) Int. Cl.: A61F 2/24, A61M 25/00, A61B 17/00

(54) **MEDICAL DEVICE ADAPTED FOR MULTIPLE IMPLANT RECAPTURES**
MEDIZINISCHE VORRICHTUNG, DIE FÜR MEHRERE IMPLANTAT-RECAPTURES ANGEPASST IST
DISPOSITIF MÉDICAL ADAPTÉ POUR DE MULTIPLES RECAPTURES D'IMPLANT

(30) Priority: 22.12.2021 US 202163292541 P
(43) Date of publication of application: 16.10.2024
(73) Proprietor: Boston Scientific Scimed, Inc., Maple Grove, MN 55311 (US)
(72) Inventor: PASCHE, Daniel William, Brooklyn Center, Minnesota 55430 (US); OST, Austin Farrell, Eden Prairie, Minnesota 55346 (US); VIOLA, Daniel James, Minneapolis, Minnesota 55406 (US)
(74) Representative: Peterreins Schley
(86) International application number: PCT/US2022/053671
(87) International publication number: WO 2023/122184

(56) References cited:
- EP-A1- 3 162 405
- WO-A1-2011/133792
- WO-A1-2016/065023
- WO-A1-2021/250980
- WO-A2-2013/119332
- JP-A- 2014 097 090
- JP-A- 2020 156 974
- US-A1- 2009 254 165

## Description

### TECHNICAL FIELD

The present disclosure pertains to medical devices, and methods for manufacturing and using medical devices. More particularly, the disclosure is directed to a medical device that is adapted for multiple deployments and recaptures of an implant.

### BACKGROUND

A wide variety of medical devices have been developed for medical use, for example, for use in accessing body cavities and interacting with fluids and structures in body cavities. Some of these devices may include guidewires, catheters, pumps, motors, controllers, filters, grinders, needles, valves, and delivery devices and/or systems used for delivering such devices. These devices are manufactured by any one of a variety of different manufacturing methods and may be used according to any one of a variety of methods. Of the known medical devices and methods, each has certain advantages and disadvantages.

EP 3 162 405 A1 relates to a catheter which comprises a catheter body having a lumen, a resin front end tip having a lumen communicating with the lumen of the catheter body and attached to the front end of the catheter body, and a coil body and/or a braid arranged at the catheter body and the front end tip along the lumen. A reinforcement body covering the coil body and/or the braid is provided at the frontend tip.

WO 2021/250980 A1 relates to a catheter which is provided with a shaft having a lumen extending from the distal end to the proximal end. The shaft has: a reinforcing body provided with a plurality of wire materials that are disposed on at least a portion between the inner surface of the shaft, which forms the lumen, and the outer surface of the shaft, and are woven into a tubular shape; and at least one X-ray opaque marker disposed between the reinforcing body and the outer surface. An outer diameter difference, which is the difference between the outer diameter of the shaft at the portion at which the marker is disposed and the outer diameter of the shaft at a portion adjacent to the marker in the axial direction of the shaft, is smaller than twice the thickness of the marker.

### SUMMARY

The invention is defined in the appended claims. Aspects, embodiments and examples disclosed herein which do not fall within the scope of the appended claims do not form part of the invention and are merely provided for illustrative purposes.

This disclosure provides design, material, manufacturing method, and use alternatives for medical devices. According to the invention, a medical device includes an elongate shaft and a rigid member that is secured relative to the elongate shaft. The elongate shaft includes an inner polymeric liner that defines a lumen extending therethrough, an outer polymeric sheath, and a reinforcing braid that extends within the outer polymeric sheath. The reinforcing braid includes a plurality of wire ends that extend beyond a distal edge of the rigid member.

According to the invention, a distal region of the elongate shaft is adapted for delivering and/or recapturing an implant, and the rigid member is disposed within the distal region of the elongate shaft.

Additionally, the plurality of wire ends may extend distally from the distal edge of the rigid member.

Additionally, the plurality of wire ends may also extend radially outwardly from the distal edge of the rigid member.

According to the invention, the plurality of wire ends form ramps that are adapted to keep the implant, when recaptured, from contacting the distal edge of the rigid member, even if the inner polymeric liner is damaged.

Additionally, the inner polymeric liner being damaged may include the inner polymeric liner delaminating and exposing the distal edge of the rigid member to an interior of the lumen extending through the inner polymeric liner.

Additionally, the plurality of wire ends may extend radially outwardly from the distal edge of the rigid member.

Additionally, the plurality of wire ends that extend radially outwardly from the distal edge of the rigid member may form ramps that are adapted to keep the implant, when recaptured, from contacting the distal edge of the rigid member, even if the inner polymeric liner is damaged.

Additionally, the rigid member may include a metallic member.

Additionally, the rigid member may include a polymeric member.

Additionally, the rigid member may include a section of hypotube.

Additionally, the rigid member may include a marker band.

As another example, a device is adapted for delivering and/or recapturing an implant. The device includes an elongate shaft and a marker band that is secured relative to the elongate shaft. The elongate shaft includes an inner polymeric liner defining a lumen extending therethrough, an outer polymeric sheath, and a reinforcing braid extending within the outer polymeric sheath. The reinforcing braid includes a plurality of wire ends that extend beyond a distal edge of the marker band, the plurality of wire ends adapted to keep the implant, when retrieved, from contacting the distal edge of the marker band, even if the inner polymeric liner is damaged.

Alternatively or additionally, the plurality of wire ends may extend distally from the distal edge of the marker band.

Alternatively or additionally, the plurality of wire ends may also extend radially outwardly from the distal edge of the marker band.

Alternatively or additionally, the plurality of wire ends may extend radially outwardly and not distally from the distal edge of the marker band.

As another example, a medical device includes an elongate shaft having a distal region. The distal region of the elongate shaft includes an inner polymeric liner defining a lumen adapted to accommodate the implant therein, an outer polymeric sheath, a reinforcing braid extending within the outer polymeric sheath, and a marker band disposed between the inner polymeric layer and the outer polymeric sheath. The reinforcing braid includes a plurality of wire ends that extend beyond the marker band, the plurality of wire ends adapted to keep the implant, when recaptured, from contacting the marker band, even if the inner polymeric liner is damaged.

Alternatively or additionally, the plurality of wire ends may extend distally from the marker band.

Alternatively or additionally, the plurality of wire ends may also extend radially outwardly from the marker band.

Alternatively or additionally, the plurality of wire ends may extend radially outwardly and not distally from the marker band.

The above summary of some embodiments is not intended to describe each disclosed embodiment or every implementation of the present disclosure. The Figures, and Detailed Description, which follow, more particularly exemplify these embodiments.

### BRIEF DESCRIPTION OF THE DRAWINGS

The invention may be more completely understood in consideration of the following detailed description of various embodiments of the invention in connection with the accompanying drawings, in which:
Figure 1 is a side view of an illustrative medical device shown with an implant partially deployed;
Figure 2 is a side view of a distal region forming a part of the illustrative medical device of Figure 1;
Figure 3 is a cross-sectional view taken along line 3-3 of Figure 2;
Figure 4 is a cross-sectional view taken along line 4-4 of Figure 3;
Figure 5 is a side view of another distal region forming a part of the illustrative medical device of Figure 1;
Figure 6 is a cross-sectional view taken along line 6-6 of Figure 5;
Figure 7 is a cross-sectional view taken along line 7-7 of Figure 6;
Figure 8 is a side view of another distal region forming a part of the illustrative medical device of Figure 1; and
Figure 9 is a side view of another distal region forming a part of the illustrative medical device of Figure 1.

While the disclosure is amenable to various modifications and alternative forms, specifics thereof have been shown by way of example in the drawings and will be described in detail. It should be understood, however, that the intention is not to limit the disclosure to the particular embodiments described. On the contrary, the intention is to cover all modifications, equivalents, and alternatives falling within the scope of the disclosure.

### DESCRIPTION

For the following defined terms, these definitions shall be applied, unless a different definition is given in the claims or elsewhere in this specification.

All numeric values are herein assumed to be modified by the term "about," whether or not explicitly indicated. The term "about" generally refers to a range of numbers that one of skill in the art would consider equivalent to the recited value (i.e., having the same function or result). In many instances, the terms "about" may include numbers that are rounded to the nearest significant figure.

The recitation of numerical ranges by endpoints includes all numbers within that range (e.g. 1 to 5 includes 1, 1.5, 2, 2.75, 3, 3.80, 4, and 5).

As used in this specification and the appended claims, the singular forms "a", "an", and "the" include plural referents unless the content clearly dictates otherwise. As used in this specification and the appended claims, the term "or" is generally employed in its sense including "and/or" unless the content clearly dictates otherwise.

The following detailed description should be read with reference to the drawings in which similar elements in different drawings are numbered the same. The drawings, which are not necessarily to scale, depict illustrative embodiments and are not intended to limit the scope of the invention.

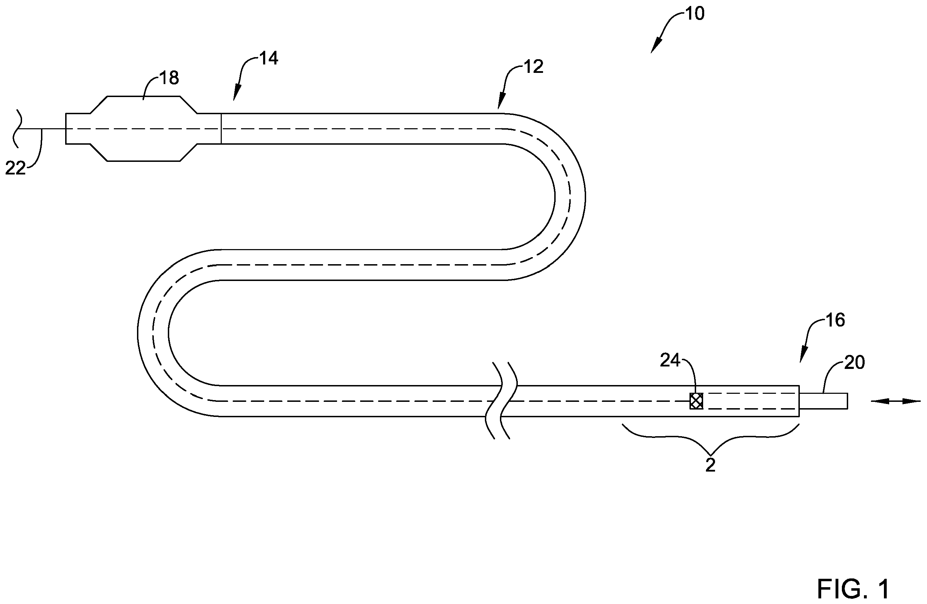

Figure 1 is a side view of an illustrative medical device 10. The medical device 10 has an elongate shaft 12 extending from a proximal region 14 to a distal region 16. As will be discussed, the elongate shaft 12 may include multiple layers made of varying materials. In some cases, the elongate shaft 12 may have a similar construction all along the elongate shaft 12, from the proximal region 14 to the distal region 16. In some cases, the distal region 16 may have a construction that is different from the construction of the proximal region 14, for example.

In some cases, the proximal region 14 may include a proximal hub 18, although this is not required in all cases. In some instances, the proximal region 14 may include additional components (not shown), including but not limited to additional hubs or fluid ports and actuatable members useable for deploying and/or retrieving an implant such as an implant 20. A portion of the implant 20 still within the distal region 16 of the elongate shaft 12 is shown in phantom, while a portion of the implant 20 that has moved distally out of the distal region 16 of the elongate shaft 12 is shown in solid line.

The implant 20 may generically represent any of a variety of different implantable medical devices that can be delivered within a device such as the medical device 10. For example, the implant 20 may represent a stent, a coil, a clip, a transcatheter valve implant such as an aortic valve implant, a mitral valve implant, a tricuspid valve implant, a filter, a snare, a balloon, and other medical devices. In some instances, the implant 20 may be a left atrial appendage (LAA) implant such as the WATCHMAN^{™} commercially available from Boston Scientific.

A deployment member 22 extends through the elongate shaft 12 and includes an engagement member 24 at a distal end thereof. A portion of the deployment member 22 extending through the elongate shaft 12 is shown in phantom, while a portion of deployment member 22 extending proximally from the elongate shaft 12 is shown in solid line. The engagement member 24 may be considered as being adapted to releasably engage the implant 20 and to continue engaging the implant 20 through one or more deployments. If an initial deployment does not yield an appropriate location or orientation of the implant 20, the implant 20 may be recaptured, repositioned and redeployed, particularly when the engagement member 24 retains a connection with the implant 20.

Once the implant 20 has been determined to be disposed in an appropriate position and orientation, for example, the engagement member 24 may be adapted to disengage the implant 20 and thus release the implant 20. In some cases, the implant 20 may never be released from the engagement member 24, and may remain attached to the engagement member 24 during use of the implant 20. After temporary use of the implant 20, the implant 20 as well as the medical device 10 may be removed from the patient.

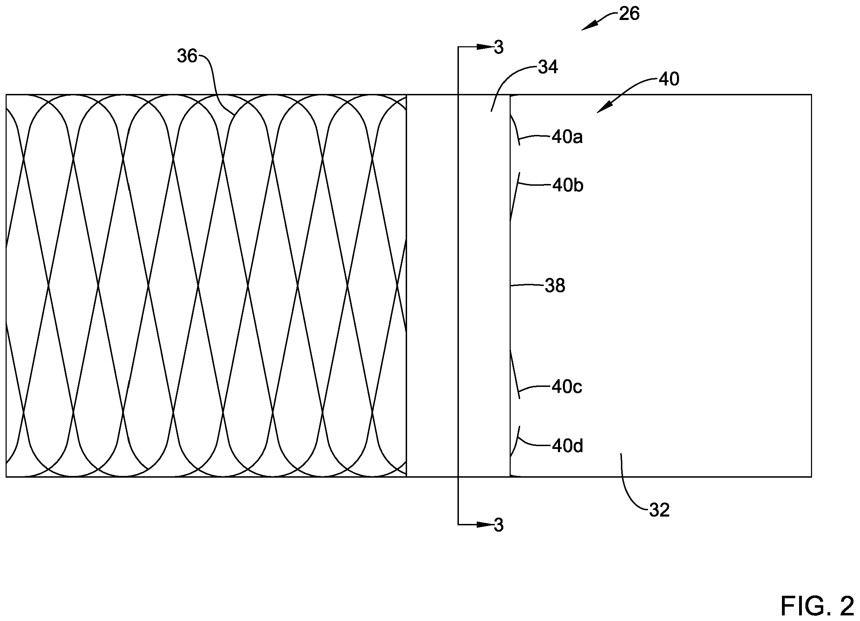

In some cases, repeated deployment and recapture of the implant 20, particularly while attempting to optimize placement of the implant 20, can stress components within the distal region 16 of the elongate shaft 12. Figures 2 through 8 provide illustrative but non-limiting examples of possible constructions for the distal region 16 of the elongate shaft 12 that can reduce or eliminate possible damage to the implant 20 even if portions of the distal region 16 of the elongate shaft 12 become damaged, or even delaminate.

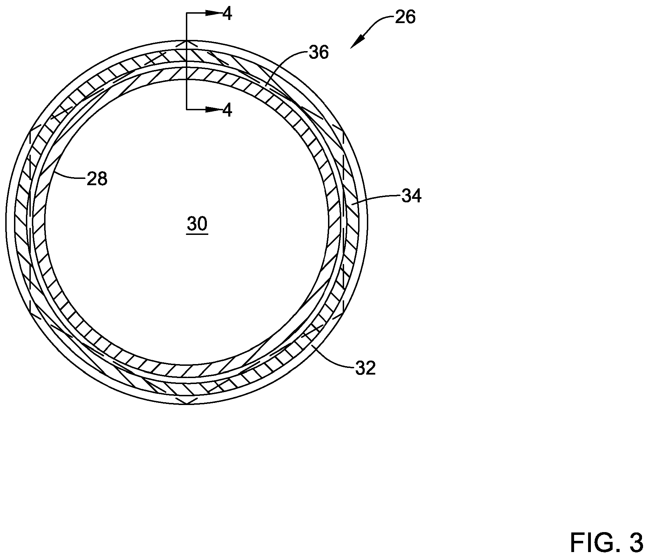

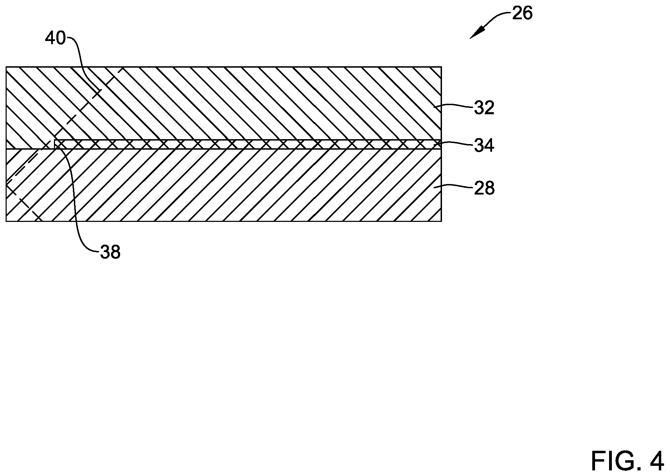

Figure 2 is a side view of a distal region 26 while Figure 3 is a cross-sectional view taken along the line 3-3 of Figure 2 and Figure 4 is a cross-sectional view taken along the line 4-4 of Figure 3. The distal region 26 may be considered as an example of the distal region 16, and can be used as part of the medical device 10. The distal region 26 is a multi-layer construction, including an inner polymeric liner 28 defining a lumen 30 extending therethrough and an outer polymeric sheath 32. In some cases, the inner polymeric liner 28 may include polytetrafluoroethylene (PTFE). In some cases, the outer polymeric sheath 32 may include polyether block amides (PEBA). In some instances, the outer polymeric sheath 32 may include any of a variety of polymeric materials.

The distal region 26 includes a rigid member 34 that in some instances represents a marker band in order to provide visualization of the distal region 26 during use of the medical device 10. In some cases, as shown, the rigid member 34 is disposed between the inner polymeric liner 28 and the outer polymeric sheath 32. The rigid member 34 may be metallic or polymeric. In cases in which the rigid member 34 serves as a marker band, the rigid member 34 may be formed of a material such as tungsten, a platinum/iridium alloy, tantalum and tantalum alloys, other metals and alloys having radiopaque properties, or stainless steel.

The outer polymeric sheath 32 includes a braid 36. Figure 2 may be considered as being a partial cutaway view in which the braid 36 is visible. The braid 36 may have any of a variety of different braiding patterns, for example, and may be formed of a variety of metallic or even polymeric materials. The braid 36 may have any number of braid wires that are woven together to form the braid 36. Suitable materials for forming the braid 36 include stainless steel, Nitinol^{™}, and Vectran^{™}, which is a liquid crystal polymer.

The braid 36 extends distally beyond a distal edge 38 of the rigid member 34. The braid 36 includes a number of wire ends 40, individually labeled here as 40a, 40b, 40c and 40d. This is merely illustrative, as the braid 36 may have a greater number of wire ends 40 extending distally beyond the distal edge 38 of the rigid member 34.

In some cases, as shown for example in Figure 3, the wire ends 40 are not tacked down to the inner polymeric liner 28, but instead are allowed to extend tangent to an inner diameter of the rigid member 34 and the wire ends 40 extend distally beyond the rigid member 34. The wire ends 40 do not curve along the inner polymeric liner 28, as other constructions may provide.

As a result, and as shown in Figure 4, the wire ends 40 extend into the outer polymeric sheath 32. This creates a ramp-like effect that guides the implant 20 past the distal edge 38 of the rigid member 34. It will be appreciated that in some cases, the edges of the rigid member 34, including the distal edge 38 of the rigid member 34, may be sharp enough to otherwise cause damage to the implant 20 if the implant 20 were to impact the distal edge 38 of the rigid member 34.

Accordingly, by constructing the distal region 26 in this fashion, with the wire ends 40 extending distally beyond the distal edge 38 of the rigid member 34, it is possible to provide the implant 20 with protection against possible damage that could otherwise be caused by delamination or other damage to the inner polymeric liner 28 that exposes the distal edge 38 of the rigid member 34. The wire ends 40 are adapted to extend tangent to the inner diameter of the rigid member 34, rather than curving along the inner polymeric liner 28.

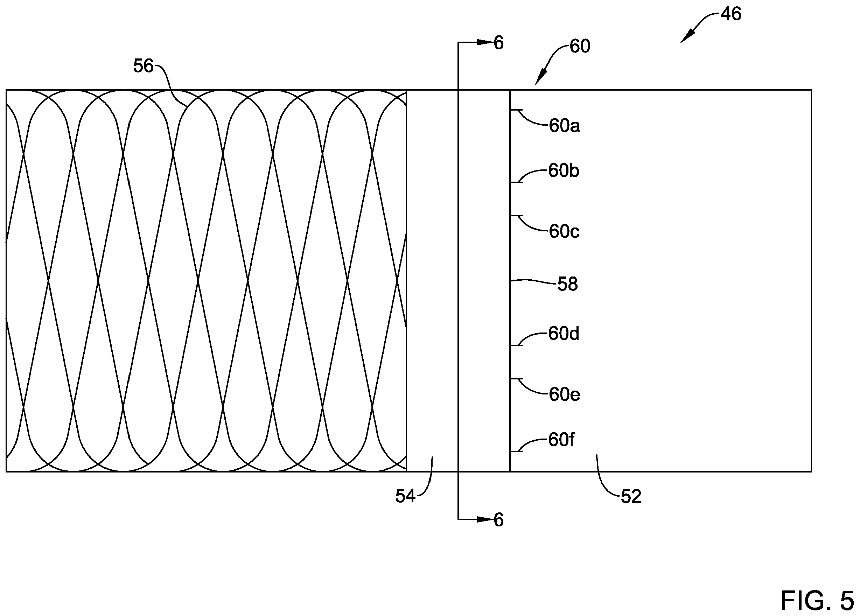

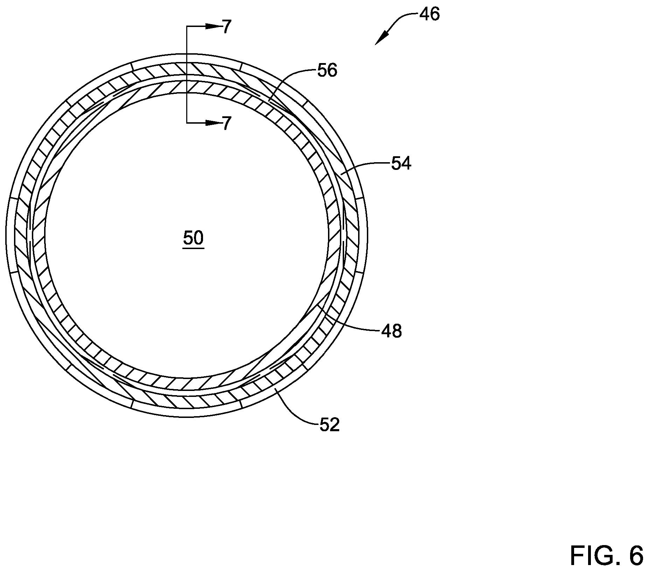

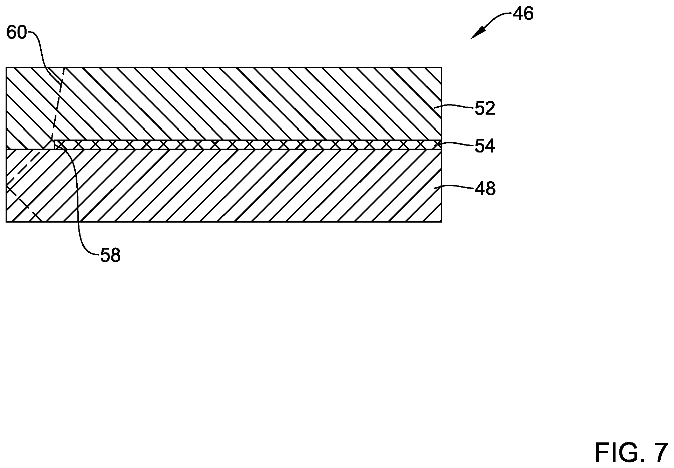

Figure 5 is a side view of a distal region 46 while Figure 6 is a cross-sectional view taken along the line 6-6 of Figure 5 and Figure 7 is a cross-sectional view taken along the line 7-7 of Figure 6. The distal region 46 may be considered as an example of the distal region 16, and can be used as part of the medical device 10. The distal region 46 is a multi-layer construction, including an inner polymeric liner 48 defining a lumen 50 extending therethrough and an outer polymeric sheath 52. The distal region 46 includes a rigid member 54 that in some instances represents a marker band in order to provide visualization of the distal region 46 during use of the medical device 10. In some cases, as shown, the rigid member 54 is disposed between the inner polymeric liner 48 and the outer polymeric sheath 52.

The outer polymeric sheath 52 includes a braid 56. Figure 5 may be considered as being a partial cutaway view in which the braid 56 is visible. The braid 56 may have any of a variety of different braiding patterns, for example, and may be formed of a variety of metallic or even polymeric materials. The braid 56 may have any number of braid wires that are woven together to form the braid 56.

The braid 56 extends distally beyond a distal edge 58 of the rigid member 54. The braid 56 includes a number of wire ends 60, individually labeled here as 60a, 60b, 60c, 60d, 60e and 60f. This is merely illustrative, as the braid 56 may have a greater number of wire ends 60 extending distally beyond the distal edge 58 of the rigid member 54.

In some cases, as shown for example in Figure 5, the wire ends 60 are not tacked down to the inner polymeric liner 48, but instead are allowed to extend radially outwardly through the outer polymeric sheath 52. The wire ends 60 do not curve along the inner polymeric liner 48, as other constructions may provide.

As a result, and as shown in Figure 7, the wire ends 60 extend into the outer polymeric sheath 52. The wire ends 60 together form a larger radius near the distal edge 58 of the rigid member 54, thereby protecting the implant 20 from the distal edge 58 of the rigid member 54. It will be appreciated that in some cases, the edges of the rigid member 54, including the distal edge 58 of the rigid member 54, may be sharp enough to otherwise cause damage to the implant 20 if the implant 20 were to impact the distal edge 58 of the rigid member 54.

Accordingly, by constructing the distal region 46 in this fashion, with the wire ends 60 extending distally beyond the distal edge 58 of the rigid member 54, it is possible to provide the implant 20 with protection against possible damage that could otherwise be caused by delamination or other damage to the inner polymeric liner 48 that exposes the distal edge 58 of the rigid member 54.

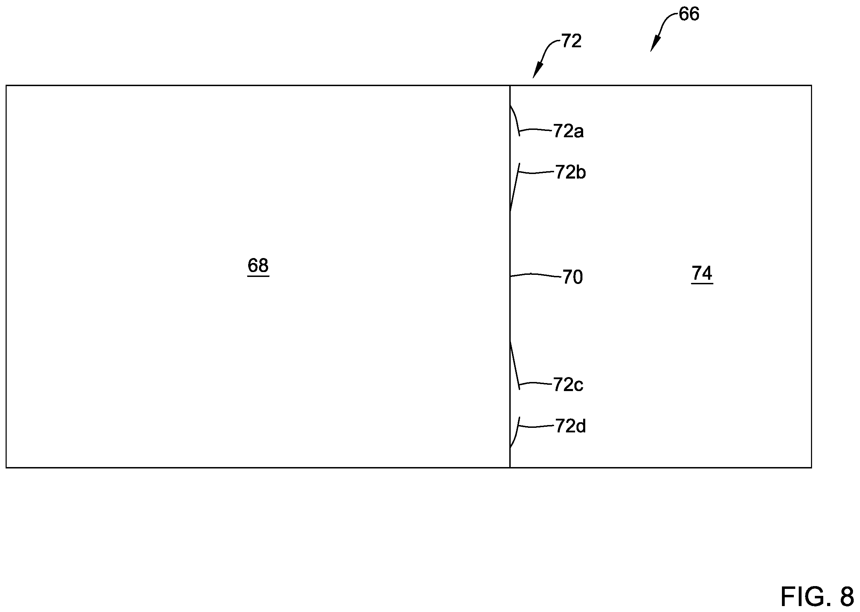

Figure 8 is a side view of a distal region 66. The distal region 66 may be considered as an example of the distal region 16, and can be used as part of the medical device 10. The distal region 66 may be a multi-layer construction, such as shown in Figures 3 and 6, for example. The distal region 66 includes a rigid member 68 that is larger than the rigid member 34 shown in Figures 2-7. In some cases, the rigid member 68 represents a section of a hypotube, and may in some cases be micromachined. The rigid member 68 includes a distal edge 70. The distal region 66 includes an outer polymeric sheath 74.

The braid, which is not visible in this view, may have any of a variety of different braiding patterns, for example, and may be formed of a variety of metallic or even polymeric materials. The braid may have any number of braid wires that are woven together to form the braid. The braid terminates distally with a number of wire ends 72, individually labeled as 72a, 72b, 72c and 72d. This is merely illustrative, as the braid may have a greater number of wire ends 72 extending distally beyond the distal edge 70 of the rigid member 68.

It will be appreciated that the arrangement of the wire ends 72 is similar to that of the wire ends 40 shown in Figure 2, and that as a result, the wire ends 72 create a ramp-like effect that guides the implant 20 past the distal edge 70 of the rigid member 68. It will be appreciated that in some cases, the edges of the rigid member 68, including the distal edge 70 of the rigid member 68, may be sharp enough to otherwise cause damage to the implant 20 if the implant 20 were to impact the distal edge 70 of the rigid member 68.

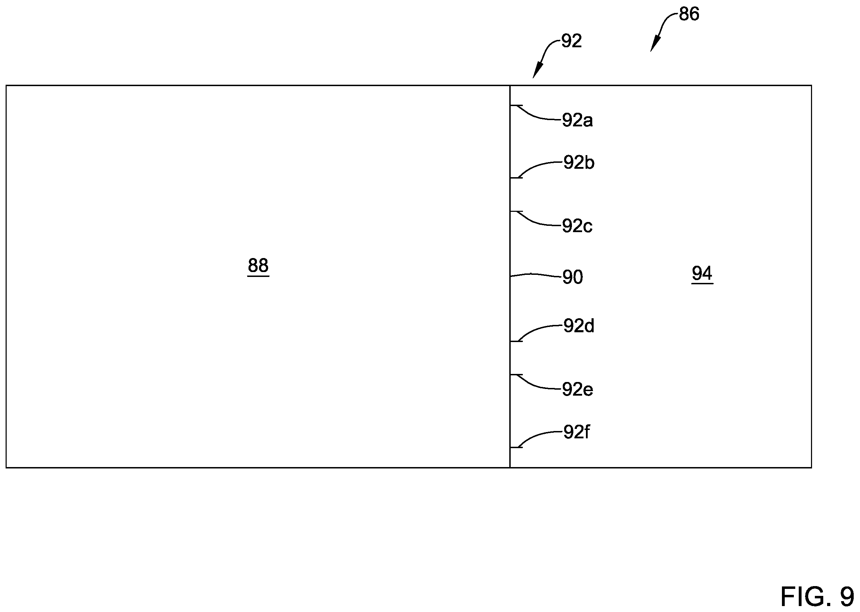

Figure 9 is a side view of a distal region 86. The distal region 86 may be considered as an example of the distal region 16, and can be used as part of the medical device 10. The distal region 66 may be a multi-layer construction, such as shown in Figures 3 and 6, for example. The distal region 86 includes a rigid member 88 that is larger than the rigid member 34 shown in Figures 2-7. In some cases, the rigid member 88 represents a section of a hypotube, and may in some cases be micromachined. The rigid member 88 includes a distal edge 90. The distal region 86 includes an outer polymeric sheath 94.

The braid, which is not visible in this view, may have any of a variety of different braiding patterns, for example, and may be formed of a variety of metallic or even polymeric materials. The braid may have any number of braid wires that are woven together to form the braid. The braid terminates distally with a number of wire ends 92, individually labeled as 92a, 92b, 92c, 92d, 92e and 92f. This is merely illustrative, as the braid may have a greater number of wire ends 92 extending distally beyond the distal edge 90 of the rigid member 88.

It will be appreciated that the arrangement of the wire ends 92 is similar to that of the wire ends 60 shown in Figure 5, and as a result, the wire ends 92 together form a larger radius near the distal edge 90 of the rigid member 88, thereby protecting the implant 20 from the distal edge 90 of the rigid member 88. It will be appreciated that in some cases, the edges of the rigid member 88, including the distal edge 90 of the rigid member 88, may be sharp enough to otherwise cause damage to the implant 20 if the implant 20 were to impact the distal edge 90 of the rigid member 88.

The devices described herein may be formed of a variety of different metallic and polymeric materials. Suitable metallic materials can include stainless steel and Nitinol. Additional examples of suitable polymers include but are not limited to polytetrafluoroethylene (PTFE), ethylene tetrafluoroethylene (ETFE), fluorinated ethylene propylene (FEP), polyoxymethylene (POM, for example, DELRIN^{®} available from DuPont), polyether block ester, polyurethane (for example, Polyurethane 85A), polypropylene (PP), polyvinylchloride (PVC), polyether-ester (for example, ARNITEL^{®} available from DSM Engineering Plastics), ether or ester based copolymers (for example, butylene/poly(alkylene ether) phthalate and/or other polyester elastomers such as HYTREL^{®} available from DuPont), polyamide (for example, DURETHAN^{®} available from Bayer or CRISTAMID^{®} available from Elf Atochem), elastomeric polyamides, block polyamide/ethers, polyether block amide (PEBA, for example available under the trade name PEBAX^{®}), ethylene vinyl acetate copolymers (EVA), silicones, polyethylene (PE), Marlex^{®} high-density polyethylene, Marlex^{®} low-density polyethylene, linear low density polyethylene (for example REXELL^{®}), polyester, polybutylene terephthalate (PBT), polyethylene terephthalate (PET), polytrimethylene terephthalate, polyethylene naphthalate (PEN), polyetheretherketone (PEEK), polyimide (PI), polyetherimide (PEI), polyphenylene sulfide (PPS), polyphenylene oxide (PPO), poly paraphenylene terephthalamide (for example, KEVLAR^{®}), polysulfone, nylon, nylon-12 (such as GRILAMID^{®} available from EMS American Grilon), perfluoro(propyl vinyl ether) (PFA), ethylene vinyl alcohol, polyolefin, polystyrene, epoxy, polyvinylidene chloride (PVdC), poly(styrene-b-isobutylene-b-styrene) (for example, SIBS and/or SIBS 50A), polycarbonates, ionomers, biocompatible polymers, other suitable materials, or mixtures, combinations, copolymers thereof, polymer/metal composites, and the like. Additional materials include liquid crystal polymers.

It should be understood that this disclosure is, in many respects, only illustrative. Changes may be made in details, particularly in matters of shape, size, and arrangement of steps without exceeding the scope of the disclosure. This may include, to the extent that it is appropriate, the use of any of the features of one example embodiment being used in other embodiments. The invention's scope is, of course, defined in the language in which the appended claims are expressed.

## Claims

1. A medical device (10), comprising:
an elongate shaft (12) comprising:
an inner polymeric liner (28, 48) defining a lumen (30, 50) extending therethrough;
an outer polymeric sheath (32, 52, 74, 94); and
a reinforcing braid (36, 56) extending within the outer polymeric sheath (32, 52, 74, 94); and
a rigid member (34, 54, 68, 88) secured relative to the elongate shaft (12);
wherein the reinforcing braid (36, 56) includes a plurality of wire ends (40, 60, 72, 92) that extend beyond a distal edge (38, 58, 70, 90) of the rigid member (34, 54, 68, 88),
wherein a distal region (16, 26, 46, 66, 86) of the elongate shaft (12) is adapted for delivering and/or recapturing an implant (20), and the rigid member (34, 54, 68, 88) is disposed within the distal region (16, 26, 46, 66, 86) of the elongate shaft (12),
wherein the plurality of wire ends (40, 60, 72, 92) form ramps that are adapted to keep the implant (20), when recaptured, from contacting the distal edge (38, 58, 70, 90) of the rigid member (34, 54, 68, 88), even if the inner polymeric liner (28, 48) is damaged.

2. The medical device of claim 1, wherein the plurality of wire ends (40, 60, 72, 92) extend radially outwardly from the distal edge (38, 58, 70, 90) of the rigid member (34, 54, 68, 88).

3. The medical device of claim 2, wherein the plurality of wire ends (40, 60, 72, 92) that extend radially outwardly from the distal edge (38, 58, 70, 90) of the rigid member (34, 54, 68, 88) form ramps that are adapted to keep the implant (20), when recaptured, from contacting the distal edge (38, 58, 70, 90) of the rigid member (34, 54, 68, 88), even if the inner polymeric liner (28, 48) is damaged.

4. The medical device of any one of claims 1 to 3, wherein the rigid member (34, 54, 68, 88) comprises a section of hypotube.

5. The medical device of any one of claims 1 to 4, wherein the rigid member (34, 54, 68, 88) comprises a marker band.

6. The medical device of any of claim 1 to 5, wherein the rigid member (34, 54, 68, 88) is disposed between the inner polymeric liner (28, 48) and the outer polymeric sheath (32, 52, 74, 94).

7. The medical device of any of claim 1 to 6, wherein the rigid member (32, 52, 74, 94) is metallic or polymeric.

8. The medical device of any of claim 1 to 7, wherein the braid (36, 56) is formed of metallic or polymeric materials.

9. The medical device of any of claim 1 to 8, wherein the wire ends (40, 60, 72, 92) extend into the outer polymeric sheath (32, 52, 74, 94).

10. The medical device of any of claim 1 to 9, wherein the wire ends (40, 60, 72, 92) extend radially outward through the outer polymeric sheath (32, 52, 74, 94).

## Patentansprüche

1. Medizinische Vorrichtung (10) umfassend:
einen langgestreckten Schaft (12) umfassend:
eine innere Polymerauskleidung (28, 48), die ein hindurch verlaufendes Lumen (30, 50) definiert;
eine äußere Polymerhhülle (32, 52, 74, 94); und
ein Verstärkungsgeflecht (36, 56), das innerhalb der äußeren Polymerhülle (32, 52, 74, 94) verläuft; und
ein starres Element (34, 54, 68, 88), das relativ zu dem langgestreckten Schaft (12) befestigt ist;
wobei das Verstärkungsgeflecht (36, 56) eine Vielzahl von Drahtenden (40, 60, 72, 92) umfasst, die sich über eine distale Kante (38, 58, 70, 90) des starren Elements (34, 54, 68, 88) hinaus erstrecken,
wobei ein distaler Bereich (16, 26, 46, 66, 86) des langgestreckten Schafts (12) zum Zuführen und/oder Wiederaufnehmen eines Implantats (20) ausgelegt ist und das starre Element (34, 54, 68, 88) innerhalb des distalen Bereichs (16, 26, 46, 66, 86) des langgestreckten Schafts (12) angeordnet ist,
wobei die Vielzahl von Drahtenden (40, 60, 72, 92) Rampen bilden, die dazu ausgelegt sind, das Implantat (20), wenn wieder aufgenommen, daran zu hindern, die distale Kante (38, 58, 70, 90) des starren Elements (34, 54, 68, 88) zu berühren, selbst wenn die innere Polymerauskleidung (28, 48) beschädigt ist.

2. Medizinische Vorrichtung nach Anspruch 1, wobei sich die Vielzahl von Drahtenden (40, 60, 72, 92) von der distalen Kante (38, 58, 70, 90) des starren Elements (34, 54, 68, 88) radial nach außen erstrecken.

3. Medizinische Vorrichtung nach Anspruch 2, wobei die Vielzahl von Drahtenden (40, 60, 72, 92), die sich von der distalen Kante (38, 58, 70, 90) des starren Elements (34, 54, 68, 88) radial nach außen erstrecken, Rampen bilden, die dazu ausgelegt sind, das Implantat (20), wenn wieder aufgenommen, daran zu hindern, die distale Kante (38, 58, 70, 90) des starren Elements (34, 54, 68, 88) zu berühren, selbst wenn die Innenauskleidung beschädigt ist (28, 48).

4. Medizinische Vorrichtung nach einem der Ansprüche 1 bis 3, wobei das starre Element (34, 54, 68, 88) einen Abschnitt von Hypotubus umfasst.

5. Medizinische Vorrichtung nach einem der Ansprüche 1 bis 4, wobei das starre Element (34, 54, 68, 88) ein Markerband umfasst.

6. Medizinische Vorrichtung nach einem der Ansprüche 1 bis 5, wobei das starre Element (34, 54, 68, 88) zwischen der inneren Polymerauskleidung (28, 48) und der äußeren Polymerhülle (32, 52, 74, 94) angeordnet ist.

7. Medizinische Vorrichtung nach einem der Ansprüche 1 bis 6, wobei das starre Element (32, 52, 74, 94) metallisch oder ein Polymer ist.

8. Medizinische Vorrichtung nach einem der Ansprüche 1 bis 7, wobei das Geflecht (36, 56) aus metallischen oder polymeren Materialien gebildet ist.

9. Medizinische Vorrichtung nach einem der Ansprüche 1 bis 8, wobei sich die Drahtenden (40, 60, 72, 92) in die äußere Polymerhülle (32, 52, 74, 94) erstrecken.

10. Medizinische Vorrichtung nach einem der Ansprüche 1 bis 9, wobei sich die Drahtenden (40, 60, 72, 92) durch die äußere Polymerhülle (32, 52, 74, 94) radial nach außen erstrecken.

## Revendications

1. Dispositif médical (10), comprenant :
une tige allongée (12) comprenant :
un revêtement polymère interne (28, 48) définissant une lumière (30, 50) s'étendant à travers celle-ci ;
une gaine polymère externe (32, 52, 74, 94) ; et
une tresse de renforcement (36, 56) s'étendant à l'intérieur de la gaine polymère externe (32, 52, 74, 94) ; et
un élément rigide (34, 54, 68, 88) solidaire par rapport à la tige allongée (12) ;
la tresse de renforcement (36, 56) comprenant une pluralité d'extrémités de fil (40, 60, 72, 92) qui s'étendent au-delà d'un bord distal (38, 58, 70, 90) de l'élément rigide (34, 54, 68, 88),
une région distale (16, 26, 46, 66, 86) de la tige allongée (12) étant adaptée pour délivrer et/ou recapturer un implant (20), et l'élément rigide (34, 54, 68, 88) étant disposé à l'intérieur de la région distale (16, 26, 46, 66, 86) de la tige allongée (12),
la pluralité d'extrémités de fil (40, 60, 72, 92) formant des rampes qui sont adaptées pour empêcher l'implant (20), lorsqu'il est recapturé, d'entrer en contact avec le bord distal (38, 58, 70, 90) de l'élément rigide (34, 54, 68, 88), même si le revêtement polymère interne (28, 48) est endommagé.

2. Dispositif médical selon la revendication 1, la pluralité d'extrémités de fil (40, 60, 72, 92) s'étendant radialement vers l'extérieur à partir du bord distal (38, 58, 70, 90) de l'élément rigide (34, 54, 68, 88).

3. Dispositif médical selon la revendication 2, la pluralité d'extrémités de fil (40, 60, 72, 92) qui s'étendent radialement vers l'extérieur à partir du bord distal (38, 58, 70, 90) de l'élément rigide (34, 54, 68, 88) formant des rampes qui sont adaptées pour empêcher l'implant (20), lorsqu'il est recapturé, d'entrer en contact avec le bord distal (38, 58, 70, 90) de l'élément rigide (34, 54, 68, 88), même si le revêtement polymère interne (28, 48) est endommagé.

4. Dispositif médical selon l'une quelconque des revendications 1 à 3, l'élément rigide (34, 54, 68, 88) comprenant une section d'hypotube.

5. Dispositif médical selon l'une quelconque des revendications 1 à 4, l'élément rigide (34, 54, 68, 88) comprenant une bande de marquage.

6. Dispositif médical selon l'une quelconque des revendications 1 à 5, l'élément rigide (34, 54, 68, 88) étant disposé entre le revêtement polymère interne (28, 48) et la gaine polymère externe (32, 52, 74, 94).

7. Dispositif médical selon l'une quelconque des revendications 1 à 6, l'élément rigide (32, 52, 74, 94) étant métallique ou polymère.

8. Dispositif médical selon l'une quelconque des revendications 1 à 7, la tresse (36, 56) étant formée de matériaux métalliques ou polymères.

9. Dispositif médical selon l'une quelconque des revendications 1 à 8, les extrémités de fil (40, 60, 72, 92) s'étendant dans la gaine polymère externe (32, 52, 74, 94).

10. Dispositif médical selon l'une quelconque des revendications 1 à 9, les extrémités de fil (40, 60, 72, 92) s'étendant radialement vers l'extérieur à travers la gaine polymère externe (32, 52, 74, 94).
